# EUROPEAN PATENT SPECIFICATION

(11) **EP 0 972 535 B1**
(45) Date of publication and mention of the grant of the patent: **28.12.2005**
(21) Application number: 99119233.7
(22) Date of filing: 11.09.1992
(51) Int. Cl.: A61M 25/00, A61M 29/02, A61F 2/06

(54) **Inflatable member having elastic expansion with limited range**
In begrenztem Bereich elastisch dehnbarer aufblasbarer Gegenstand
Objet gonflable ayant une expansion élastique limitée

(30) Priority: 12.09.1991 US 758630; 23.07.1992 US 918232
(43) Date of publication of application: 19.01.2000
(62) Divisional of application: 92115587.5
(73) Proprietor: ADVANCED CARDIOVASCULAR SYSTEMS, INC., Santa Clara California 95054 (US)
(72) Inventor: Muni, Ketan P., San Jose, CA 95136 (US); Powers, Gerard F., San Ramon, CA 94583 (US); Samson, Wilfred J., Saratoga, CA 95070 (US); Taylor, Kevin D., Colorado 95148 (US); Bagaoisan, Celto Jacinto, Newark, CA 94560 (US)
(74) Representative: Molnia, David

(56) References cited:
- EP-A- 0 364 787
- EP-A- 0 399 712
- EP-A- 0 441 516
- WO-A-90/01969
- US-A- 4 922 905
- US-A- 4 994 071

## Description

### BACKGROUND OF THE INVENTION

This invention generally relates to elongated catheters, for delivering a stent

PTCA is a widely used procedure for the treatment of coronary heart disease. In this procedure, a balloon dilatation catheter is advanced into the patient's coronary artery and the balloon on the catheter is inflated within the stenotic region of the patient's artery to open up the arterial passageway and thereby increase the blood flow therethrough. To facilitate the advancement of the dilatation catheter into the patient's coronary artery, a guiding catheter having a preshaped distal tip is first percutaneously introduced into the cardiovascular system of a patient by the Seldinger technique through the brachial or femoral arteries. The catheter is advanced until the preshaped distal tip of the guiding catheter is disposed within the aorta adjacent the ostium of the desired coronary artery. The guiding catheter is twisted or torqued from the proximal end, which extends out of the patient, to guide the distal tip of the guiding catheter into the ostium. A balloon dilatation catheter may then be advanced through the guiding catheter into the patient's coronary artery until the balloon on the catheter is disposed within the stenotic region of the patient's artery. The balloon is inflated to open up the arterial passageway and increase the blood flow through the artery.

One type of catheter frequently used in PTCA procedures is an over-the-wire type balloon dilatation catheter. Commercially available over-the-wire type dilatation catheters include the SIMPSON ULTRA-LOW PROFILE®, the HARTZLER ACX®, the HARTZLER ACX II®, the PINKERTON .018™ and the ACS TEN™ balloon dilatation catheters sold by the assignee of the present invention, Advanced Cardiovascular Systems, Inc. (ACS). When using an over-the-wire dilatation catheter, a guidewire is usually inserted into an inner lumen of the dilatation catheter before it is introduced into the patient's vascular system and then both are introduced into and advanced through the guiding catheter to its distal tip which is seated within the ostium. The guidewire is first advanced out the seated distal tip of the guiding catheter into the desired coronary artery until the distal end of the guidewire extends beyond the lesion to be dilatated. The dilatation catheter is then advanced out of the distal tip of the guiding catheter into the patient's coronary artery, over the previously advanced guidewire, until the balloon on the distal extremity of the dilatation catheter is properly positioned across the lesion to be dilatated. Once properly positioned across the stenosis, the balloon is inflated one or more times to a predetermined size with radiopaque liquid at relatively high pressures (*e.g.*, generally 4-12 atmospheres) to dilate the stenosed region of a diseased artery. After the inflations, the balloon is finally deflated so that the dilatation catheter can be removed from the dilated stenosis to resume blood flow.

Fixed-wire type dilatation catheter systems are also utilized very frequently in PTCA procedures. This type of dilatation catheter has guidewire or guiding member secured within the catheter and it provides a low profile, *i.e*. small transverse dimensions, because there is no inner tubular member which is characteristic of commercially available over-the-wire dilatation catheters. Commercially available fixed-wire dilatation catheters include the HARTZLER EXCEL®, the HARTZLER LPS® and the SLALOM™ dilatation catheters sold by ACS.

Another type of dilatation catheter, the rapid exchange type catheter, was introduced by ACS under the trademark ACS RX® Coronary Dilatation Catheter. It is described and claimed in U.S. Patent 5,040,548 (Yock), U.S. Patent 5,061,273 (Yock), and U.S. Patent 4,748,982 (Horzewski *et al.*). This dilatation catheter has a short guidewire receiving sleeve or inner lumen extending through a distal portion of the catheter. The sleeve or inner lumen extends proximally from a first guidewire port in the distal end of the catheter to a second guidewire port in the catheter spaced proximally from the inflatable member of the catheter. A slit may be provided in the wall of the catheter body which extends distally from the second guidewire port, preferably to a location proximal to the proximal end of the inflatable balloon. The structure of the catheter allows for the rapid exchange of the catheter without the need for an exchange wire or adding a guidewire extension to the proximal end of the guidewire. This catheter has been widely praised by the medical profession and it has met with much success in the marketplace because of the advantages of its unique design.

The perfusion type dilatation catheter was another type of dilatation catheter introduced into the marketplace by ACS. This catheter, which can take the form of an over-the-wire catheter or a rapid exchange type catheter, has one or more perfusion ports proximal to the dilatation balloon in fluid communication with an guidewire receiving inner lumen extending to the distal end of the catheter. One or more perfusion ports are preferably provided in the catheter distal to the balloon which are also in fluid communication with the inner lumen extending to the distal end of the catheter. When the balloon of this dilatation catheter is inflated to dilate a stenosis, oxygenated blood in the artery or the aorta or both, depending upon the location of the dilatation catheter within the coronary anatomy, is forced to pass through the proximal perfusion ports, through the inner lumen of the catheter and out the distal perfusion ports. This provides oxygenated blood downstream from the inflated balloon to thereby prevent or minimize ischemic conditions in tissue distal to the catheter. The perfusion of blood distal to the inflated balloon allows for long term dilatations, *e.g*. 30 minutes or even several hours or more. This catheter has likewise been highly praised by the medical profession and has met with much commercial success. Commercially available perfusion type dilatation catheters include the STACK PERFUSION™ and the ACS RX PERFUSION™ Dilatation Catheters which are sold by ACS.

The balloons for prior dilatation catheters utilized in angioplasty procedures generally have been formed of relatively inelastic polymeric materials such as polyvinyl chloride, polyethylene, polyethylene terephthalate and polyolefinic ionomers. Nylon has been mentioned in the literature as an alternative inelastic material from which dilatation balloons can be made, but there has not been much commercial use of this material. The aforementioned prior art balloons are characteristically relatively inelastic so that upon inflation with inflation liquid there is relatively little expansion of the balloon with increased internal pressures, even at very elevated levels. However, when the prior art balloons were deflated, the inelastic balloon material did not shrink or contract, so as a result, the deflated profiles of the prior art balloons were relatively large. In an effort to reduce the deflated profiles of the prior art balloons made formed polyethylene, polyvinyl chloride and polyolefinic ionomers. very frequently they would be heat formed so as to wrap around inner members extending through the interior of the balloons. However, balloons formed of polyethylene terephthalate were not readily heat formed, with the result that, when a vacuum was pulled on the balloon, wings were formed which extend outwardly presenting a relatively large profile.

What has been needed and heretofore unavailable is a thin walled inflatable member for intravascular catheters which upon inflation exhibits a controlled elastic expansion but which does not expand significantly beyond a particular pressure level. The present invention satisfies these and other needs.

### SUMMARY OF THE INVENTION

This invention is directed to an inflatable member such as a balloon which exhibits upon inflation a substantial elastic expansion within a first pressure range and which is considerably less compliant at pressures beyond the first pressure range. Upon deflation, the inflatable member contracts by elastic recoil to a diameter much smaller than the inflated diameter.

Disclosed herein is an inflatable member having an expandable wall portion which at internal pressures within a first pressure range exhibits substantial elastic expansion and within a second pressure range, higher than the first pressure range, exhibits relatively little expansion.

The inflatable member of the invention is a tubular member which when inflated exhibits a relatively high rate of elastic expansion within a first range of internal pressures and a relatively low rate of expansion, i.e. is much less compliant, at pressures within a second range of internal pressures higher than the first range. In one presently preferred embodiment, during the initial stage of inflation, when the internal pressures are below the first pressure range, the inflatable member or balloon is relatively noncompliant and experiences relatively little expansion, but when the internal pressures reach the first pressure range, the inflatable member expands elastically at a relatively high rate until the pressure enters a second pressure range at which point the inflatable member becomes relatively noncompliant and the expansion rate thereof is quite low. The expansion at failure is usually less than 25% and preferably less than 10% of the maximum inflated diameter at the end of the elastic expansion. Upon deflation of the inflatable member, it contracts to a diameter much smaller than the inflated diameter by means of elastic recoil.

In the deflated condition the inflatable member of the invention preferably has outer dimensions which are essentially the same as or not much larger than adjacent portions of the catheter shaft in order to present a relatively smooth outer surface which greatly facilitates the insertion and advancement of the inflatable member through the vascular system of a patient and through stenotic region of the patient's artery. When subjected to a vacuum, the inflatable member forms very small wings or essentially no wings at all which helps the passage of the inflatable member through the patient's blood vessels and through stenoses.

The inflatable member of the invention may be formed of heat shrinkable thermoplastic material, particularly a radiation cross-linked polymer material, which has been thermally treated at a temperature of not more than about 50° C above and not more than about 50° C below the crystalline melting point of the polymer to provide the requisite expansion of the present invention. In one presently preferred embodiment the inflatable member is formed of a polyolefinic ionomer such as those sold under the trademark Surlyn® by E.I. duPont, deNemours & Co. and particularly Surlyn® 8020/IBE which is a sodium cation ionomer. Other suitable ionomers include sodium ionomers 8920 and 8940, zinc ionomer 9020 and lithium ionomers 7930 and 7940 which are all sold under the trademark Surlyn®. An inflatable member or balloon formed of this material and treated in the prescribed manner has at body temperature a radial expansion which is generally elastic and predictable at pressures within a particular first range of pressures and which is considerably less compliant at pressures above the range of pressures. In another presently preferred embodiment, the cross-linked ionomer is a zinc ionomer sold under the designation F1855 by E.I. duPont, deNemours & Co. which is a lower molecular weight variant of the Surlyn® 9020 zinc ionomer mentioned above. Other heat shrinkable polymers include high density and low density polyethylene and mixtures thereof and ethylene vinyl acetate such as EVAC® sold by duPont, deNemours & Co. may also be treated in the above manner to provide such properties. However, most thermoplastic polymers other than the ionomers, usually do not exhibit the requisite expansion characteristics at the pressure levels normally employed in angioplasty procedures, i.e. they exhibit these characteristics at much higher pressures. However, by heating the inflatable member formed of non-ionomer polymers while it is inflated, the pressure levels at which the polymer will exhibit the requisite expansion characteristics will be lowered considerably.

In one embodiment of the invention an inflatable member which has the desired elastic expansion at body temperature (37° C) is provided on the distal portion of a catheter adapted for intravascular procedures such as angioplasty. The catheter has an elongated shaft with proximal and distal extremities and an inner lumen extending therein for directing inflation fluid therethrough to the interior of the inflatable member. The inflatable member may be formed of the same material as the entire catheter shaft or only a distal portion of the shaft or it may be formed of the same or different materials and secured by suitable means such as adhesive, heat bonding or heat shrinking to the distal end of the shaft. An adapter is provided on the proximal end of the elongated shaft to direct inflation fluid through the inner lumen of the catheter shaft to the interior of the inflatable member.

The inflatable member of the invention can be used in essentially all dilatation catheters for angioplasty procedures including those described in the Background of the Invention. By heat treating only a portion of the inflatable member, *e.g*. along one side, only one side of the inflatable member will inflate. This can be advantageously used with eccentric lesions within the patient's artery. The inflatable member of the invention may also be used in other types of catheters. For example, they may be employed with atherectomy devices such as described in U.S. Reissue Patent 33, 569 to position the cutting head at a desired position within the blood vessel or they can be employed to adjust the pressure of the cutting head against the atherosclerotic mass to force more or less of the mass within the cutting chamber of the cutting head. The inflatable member of the invention can also be used in catheters such as described in U.S. Patent 4,932,959 (Songer), which have means to releasably secure a guidewire within an inner lumen within the interior of the balloon without inflating the balloon to provide increased pushability to the catheter to facilitate crossing tight or completely occluded lesions.

The relatively noncompliant nature of the balloon material at inflation pressures greater than the inflation pressures within the first pressure range provides a great degree of ensurance that the inflatable member does not over inflate within the patient's vasculature which could cause damage to the arterial wall or other body lumen in which the inflatable member is disposed. Moreover, to the extent that the balloon is inflated to pressures greater than the first pressure range, the small amount of balloon expansion which does occur can be controlled by the physician by observing the pressure of the inflation fluid within the catheter. If the physician finds that the conditions within the blood vessel require an inflatable member or balloon of a slightly greater diameter than the diameter originally contemplated, all the physician needs to do is to adjust the pressure of the inflation fluid within the interior of the balloon to a predetermined level to safely provide an inflated balloon diameter of the desired size.

For inflatable members formed of many presently available polymer systems, the first pressure range, wherein the expansion is in the elastic mode, can be very high and in some instances can be too high for the intravascular and intraluminal uses contemplated herein. It has been found that the expansion of the inflatable member can be initiated at much lower pressures by heat treating and preexpanding the inflatable member at the heat treat temperature, cooling the preexpanded member and then heat shrinking it. However, in this case the maximum pressure at which the expandable member elastically expands remains essentially the same, i.e. the rate of elastic expansion of the inflatable member decreases but the maximum pressure within the elastic pressure range does not significantly change. In this embodiment of the invention, the initial diameter of the deflated inflatable member is larger than the diameter of the inflatable member which has not been preexpanded and heat shrunk. However, upon applying a vacuum to the interior of the inflatable member in accordance with the present invention, it has been found to readily form small wings which tend to wrap around any inner tubular member to reduce the deflated profile of the catheter. The relatively small wings allow the inflatable member to expand upon inflation without applying significant shear stress to the stenotic region. There is much evidence demonstrating that high shear stress on the lesion can cause dissections which can interfere with blood flow through the arterial passageway and that high shear stress can develop an arterial lining on which restenosis is rapid.

In yet another embodiment of the invention, means are provided to heat the inflatable member after it has been inserted into a patient's vasculature or other body lumen to reduce the pressure required to inflate the inflatable member to an operable size. Suitable systems for heating the inflatable member by radio frequency energy are disclosed in copending applications Serial No. 07/351,777, filed May 15, 1989 and Serial No. 07/521,337, filed May 9, 1990.

The inflatable members of the invention may also be employed in dilatation catheters used in other body lumens such as the catheter described in copending application Serial No. 07/483,397, filed February 14, 1990 which is adapted to dilate a prostatic urethra subject to hyperplasia.

Catheters having inflatable members in accordance with the invention are used to deliver stents.

These and other advantages of the invention will become more apparent from the following detailed description thereof when taken in conjunction with the accompanying exemplary drawings.

### BRIEF DESCRIPTION OF THE DRAWINGS

FIG. 1 is an elevational view partially in section of a dilatation catheter embodying features of the invention.
FIG. 2 is a transverse cross-sectional view of the catheter shown in FIG. 1 taken along the lines 2-2.
FIG. 3 is a transverse cross-sectional view of the catheter shown in FIG. 1 taken along the lines 3-3.
FIG. 4 is an elevational view of the distal portion of the catheter shown in FIG. 1 with the inflatable section in an inflated condition.
FIG. 5 is a graphical representation of the relationship of the outer diameters of inflatable members of the invention with respect to the internal pressure.
FIG. 6 is a longitudinal cross-sectional view of the distal portion of a dilatation catheter embodying features of the invention having means to releasably secure a guidewire within the catheter.
FIG. 7 is a longitudinal cross-sectional view of a distal portion of a dilatation catheter as shown in FIG. 6 wherein the means to releasably secure a guidewire within the catheter is engaged with the guidewire.
FIG. 8 is a longitudinal cross-sectional view of a distal portion of a dilatation catheter embodying features of the invention having means to heat the inflatable section.
FIG. 9 is a transverse cross-sectional view of the distal portion of the dilatation catheter shown in FIG. 8 taken along the lines 9-9.
FIG. 10 is a transverse cross-sectional view of the distal portion of the dilatation catheter shown in FIG. 8 taken along the lines 10-10.
FIG. 11 is a longitudinal view of a distal portion of a dilatation catheter embodying features of the invention having an expandable tubular element which is capable of absorbing drugs or other therapeutic fluids mounted on the exterior of the inflatable section of the catheter.
FIG. 12 is a transverse cross-sectional view of the embodiment shown in FIG. 11 taken along the lines 12-12.
FIG. 13 is a longitudinal cross-sectional view of the embodiment in FIG. 11 with the inflatable section in an inflated condition.
FIG. 14 is a longitudinal cross-sectional view of the distal portion of a steerable, fixed-wire dilatation catheter having an inflatable section which embodies features of the invention.

### DETAILED DESCRIPTION OF THE INVENTION

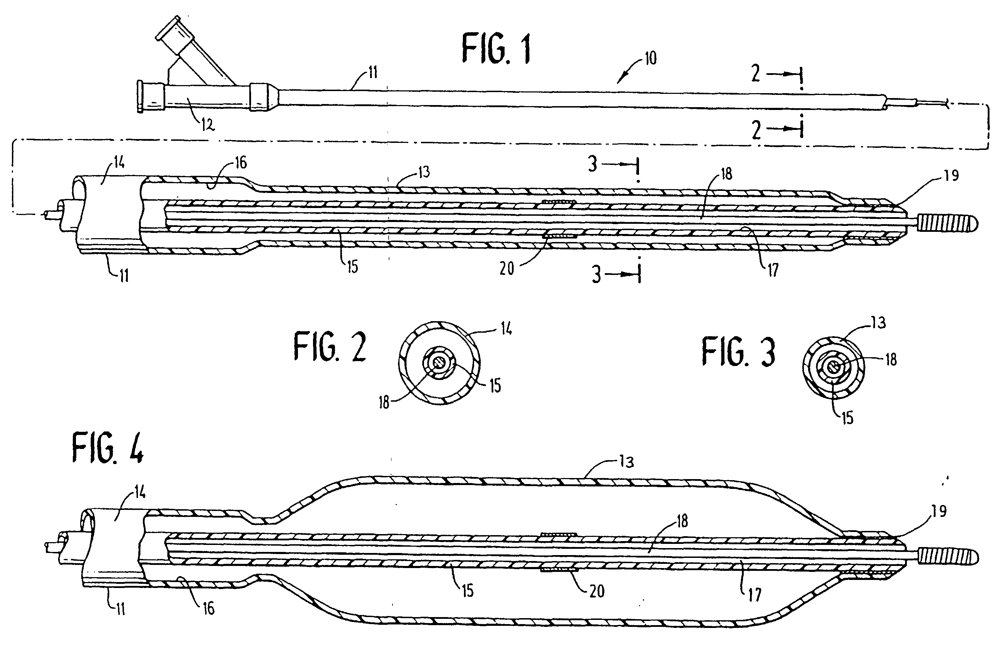

Reference is made to FIGS. 1-3 which illustrate a dilatation catheter 10. The dilatation catheter 10 generally includes a catheter shaft 11 with an inflatable tubular section 12 on the distal extremity of the catheter and an adapter 13 on the proximal end thereof and. The catheter shaft 11 has an outer tubular member 14 which is provided with the inflatable tubular section 12 and an inner tubular member 15 which is disposed within the outer tubular member 14 and defines with the outer tubular member an annular inflation lumen 16 which is adapted to direct inflation fluid from the proximal end of the catheter 10 to the interior of the inflatable section 12 of the catheter.

The inner tubular member 15 has an inner lumen 17 extending from the proximal to the distal end thereof which is adapted to receive a guidewire 18. The distal end of the inflatable portion 12 of the outer tubular member 14 is bonded by a suitable adhesive 19, e.g. a cyanoacrylate based adhesive such as Loctite® 414, to the distal end of the inner tubular member 15 to thereby seal the interior of the inflatable section 12 to prevent the loss of inflation fluid. While not shown in the drawings, a means such as one or more small passageways to vent air from the interior of the inflatable portion may be provided, such as described in U.S. Patent 4,638,805 (Powell) and U.S. Patent 4,821,722 (Miller *et al*.). A radiopaque marker 20 is provided about the portion of the inner tubular member 15 which extends through the interior of the inflatable section 12. The marker 20 is located at the midpoint of the inflatable section 12 to facilitate fluoroscopic observation thereof when the catheter is disposed within a patient's vascular system or other body lumen.

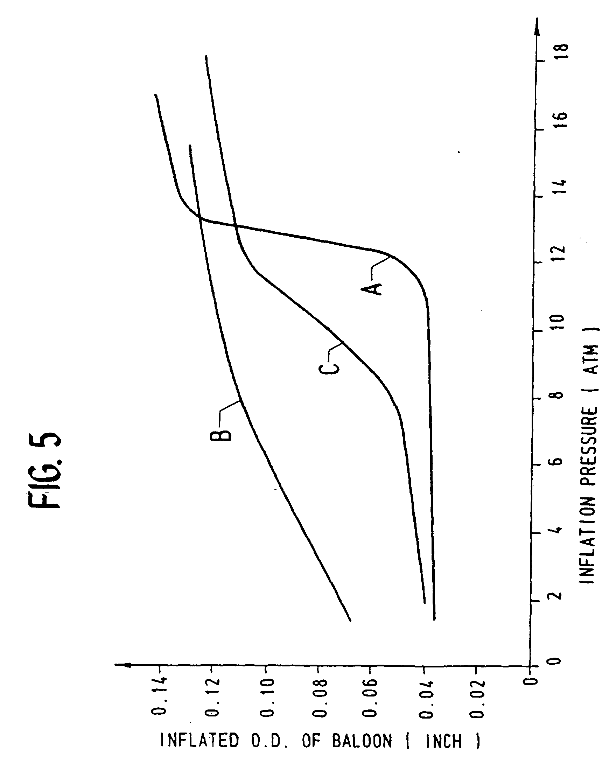

The expansion of the inflatable section 12 of the outer tubular member 14 upon the introduction of inflation fluid within the interior thereof, as described in the example below, is illustrated in FIG. 5.

The following example is given to further illustrate features of the invention. An outer tubular member 14 for a dilatation catheter 10 was prepared having a structure essentially as shown in FIGS. 1-3 and made of Surlyn® (8020 grade), a sodium ionomer supplied by the E.I. duPont, deNemours & Company. The outer tubular member 14 has an OD of about 0,94 mm (0.037) inch and an ID of about 0,64 mm (0.025 inch), *i*.*e*. a wall thickness of about 0,15mm (0.006 inch) over essentially its entire length. The outer tubular member 14 has irradiated at a level of about 45 to 55 mrads in order to cross-link essentially the entire polymeric tube. The distal portion of the polymerized tubular member which was to become the inflatable portion 12 of the catheter 10 was subjected to a thermal treatment at about 121°C (250° F) for a period of about 20 seconds while applying tension in the longitudinal direction in order to develop a significant level of longitudinal orientation in the inflatable portion. After the thermally treated inflatable section 12 of the tubular member had cooled to room temperature, the dilatation catheter 10 was assembled. The interior of the inflatable section 12 of the catheter was subjected to increasing internal pressures ranging from atmospheric to a burst pressure of about 20 atmospheres and the outer diameter of the inflatable member was determined at the various internal pressures. The relationship between the outer diameter of the inflatable section 12 and the fluid pressure within the inflatable tubular section is shown as curve A in FIG. 5. As is evident, at pressures from atmospheric to about 12 atmospheres, the change in the outer diameter of the inflatable section was negligible, *i.e*. from 0,94mm (0.037) to about 1,02 mm (0.04 inch) indicating that the material had little compliance within this pressure range. At pressures from about 12 to about 14 atmospheres the material exhibited a substantial (three-fold) elastic expansion from 0,91mm (0.036) to about 2,92 mm (0.115 inch). At internal pressures beyond the range of 12 to 14 atmospheres, the increase in the outer diameter of the inflatable section 12 was very small, *i.e*. less than about 10% of the maximum diameter within the elastic expansion range, indicating noncompliance. Even though the expansion was very small, it was essentially linear with respect to the interior pressure within the inflatable section and was therefore predictable.

When the inflatable section 12 is inflated to a larger diameter, frequently a differential develops between the length of the inflated inflatable section and the underlying portion of the inner tubular member 15, *i.e*. the outer tubular member 14 shrinks in the longitudinal direction, causing the inflated inflatable section to become deformed. To minimize or eliminate the differential, the inflatable section of the outer tubular member 14 is first formed into a balloon by subjecting this section to an internal pressure at elevated temperatures, *e.g.* from about 121°C (250° F), in a conventional manner and cooling the balloon while it is inflated, *e.g*. to room temperature, to impart heat shrinking characteristics. After cooling, the inflated inflatable portion 12 is deflated. The outer tubular member 14 is then assembled with the inner tubular member 15 and then the treated inflatable section 12 is heat shrunk onto an inner member such as the inner tubular member 15 or a mandrel (not shown) thereby eliminating or minimizing differential elongations upon the inflation of the inflatable section. Heat shrinking is effected by heating to a temperature of about 50° to about 80° C, preferably about 55° to about 65° C for at least about 5 seconds and generally not longer than about 1 hour. The heating for first forming the balloon prior to the heat shrinking thereof can be heat treatment to provide the expansion characteristics of the invention which is generally within the range of 50° C above or below the crystalline melting point of the polymer.

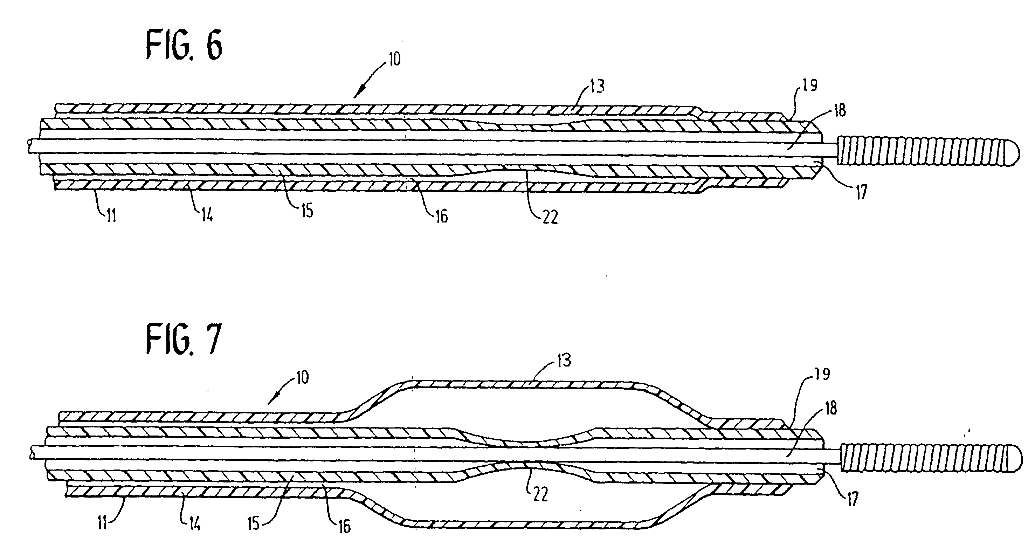

FIGS. 6 and 7 illustrate a modified embodiment of the invention having means to releasably secure a guidewire 18 within the catheter 10 so that the catheter and the guidewire can be advanced together as a unit through an artery and a stenosis within the artery as described in U.S. Patent 4,932,959 (Horzewski *et al*.). In the embodiment shown in FIGS 6 and 7, the inner tubular member 15 is provided with a thin wall section 22 which is designed to collapse onto a guidewire 18 disposed within the inner lumen 17 at a pressure less than the pressure range in which the inflatable member 12 expands elastically. In this manner, the annular inflation lumen 16 of the catheter may be subjected to a first pressure much less than the pressure range effecting elastic expansion to cause the thin wall section 22 to collapse about the guidewire 18 thereby releasably securing it within the catheter. The combined catheter and guidewire assembly has much better pushability than either the catheter or the guidewire alone so the assembly can be more easily advanced through a tight stenosis. Once the inflatable portion 12 of the dilatation catheter 10 is disposed across the stenosis, the pressure of the inflation fluid within the catheter may then be increased to a level above the threshold level to inflate the inflatable section 12 to the desired size to dilate the stenosis. After the dilatation, the pressure of the inflation fluid may be reduced to a level which allows the inflated section 13 to return to its original size. If no further dilatations are to be done, the pressure may be reduced to even lower levels to allow the thin wall section 22 of the inner tubular member 15 to return to its original position, thereby releasing the guidewire 18 therein.

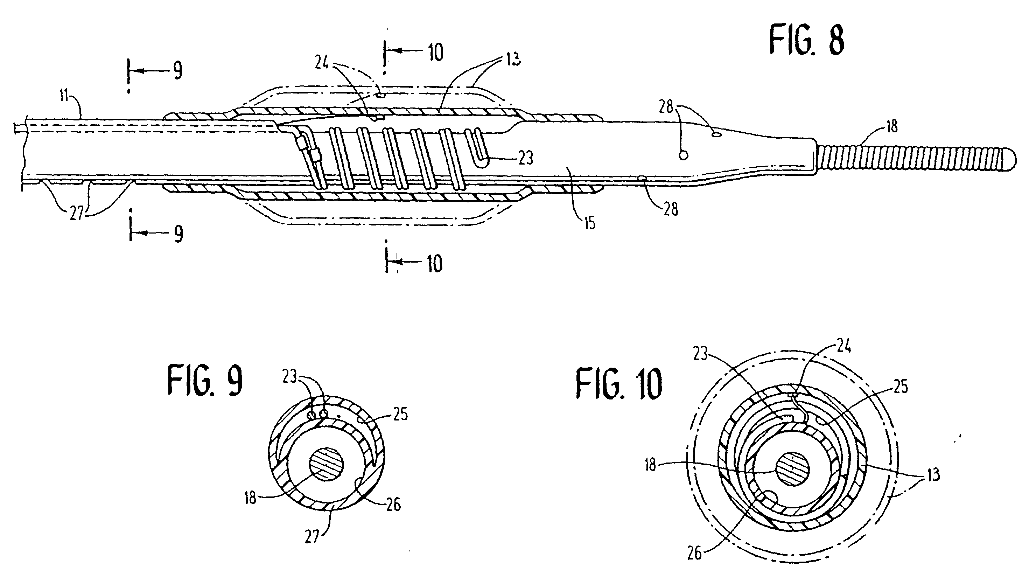

FIGS. 8-10 illustrate yet another catheter wherein the inflatable member 12 is heated while it is being inflated within the patient in order to reduce the pressure range in which substantial expansion of the inflatable member occurs in the elastic mode. In this embodiment, a heater coil 23, which is electrically connected to an electrical power source (not shown), is disposed about the inner tubular member 15 which extends through the interior of the inflatable section 12. A thermocouple 24 may be provided to sense the temperature of the inflatable section 12 or the inflation fluid therein so that the control means (not shown) may compare the temperature sensed with a desired temperature limit and adjust the electrical power from the source accordingly to control the temperature as desired. The pressure and temperature relationship with respect to the inflated diameter of the inflatable section are readily determined before the insertion of the catheter within a patient's vasculature so that the desired inflated diameter of the inflatable section can be obtained by the physician by noting the pressure and adjusting the temperature of the inflatable wall portion of the balloon or vice versa. Generally, a rise in the temperature of the wall of the inflatable section will lower the pressure range wherein there is a substantial elastic expansion of the inflatable section as shown in FIG. 5 and reduce somewhat the rate of pressure increase. Once the inflatable member has been inflated above the pressure range for elastic expansion, the temperature of the inflatable member can be allowed to return to body temperature and the angioplasty or other procedure can be completed in a conventional fashion. The catheter shaft 11 of this embodiment differs somewhat from that shown in the prior embodiments in that it has two inner lumens extending side-by-side therein, a first or inflation lumen 25 which is crescent shaped in transverse cross-section and a second or guidewire receiving lumen 26 which is circular in cross-section as shown in FIG. 9. The inflation section 13 is shown in the inflated condition in phantom in FIGS. 8 and 10. In this embodiment proximal and distal perfusion ports 27 and 28 respectively are provided so, that upon inflation of the inflatable section 13 to dilate a stenosis, oxygenated blood will flow through the proximal perfusion ports 27 into the inner lumen 17 and out the distal perfusion ports to reduce the possibility of ischemic conditions developing in tissue distal to the catheter.

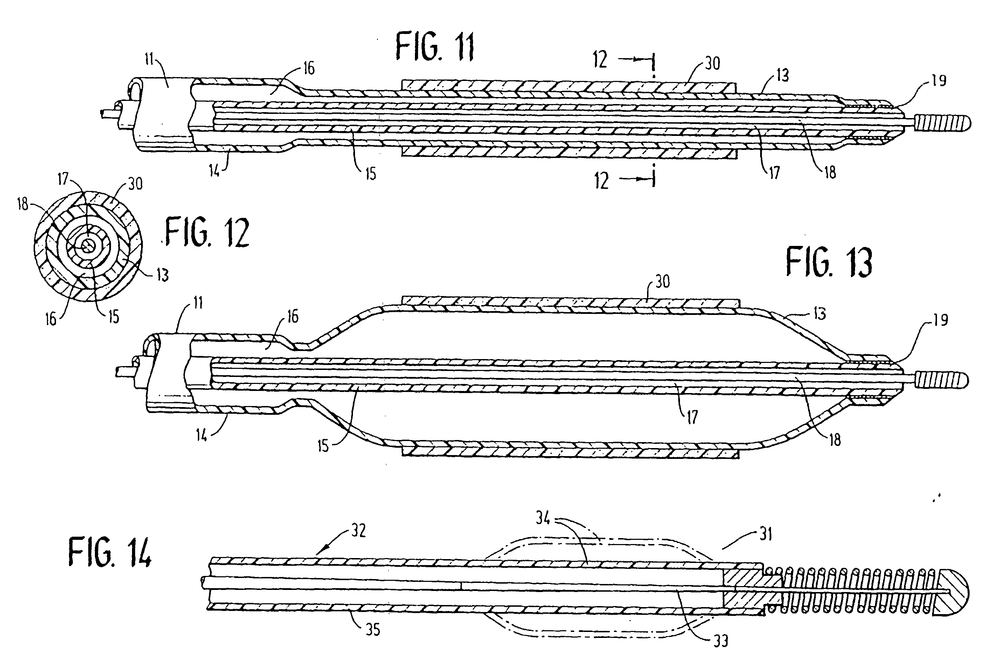

FIGS 11-13 illustrate another catheter wherein the catheter 10 is adapted to deliver drugs to a desired location within a patient's body lumen, such as a blood vessel. In the embodiment shown a tubular element 30 is capable of absorbing liquid drugs or therapeutic fluids is disposed about the inflatable section 12 of the invention. Inflation of the inflatable section 12 increases the diameter of the tubular element 30, compressing the wall thereof and driving out fluid absorbed therein to deliver the drug or therapeutic fluid to the desired location within the patient's body lumen. In an alternate embodiment (not shown) a second inflatable member having a plurality of small apertures through the wall thereof is disposed about the inflatable section 12 with liquid drugs or therapeutic fluids disposed between the inflatable section 12 and the second inflatable member so that inflation of the inflatable section will drive the liquid through the apertures in the outer second inflatable member. The number and size of the apertures in the wall of the outer balloon wall are determined for the most part by the nature of the therapeutic fluid, *e.g*. viscosity and the like, and the amount of fluid to be delivered and the rate at which it is to be delivered.

A steerable, fixed wire dilatation catheter 31 is depicted in FIG. 14 which has a tubular shaft 32 having a proximal portion (not shown) formed of stainless steel or superelastic Nitinol hypotubing with a guiding member 33 secured by its proximal end to the distal portion of the hypotubing (not shown). An inflatable member 34 embodying features of the invention is disposed about the guiding member 33 with the distal end of the inflatable section 34 being sealingly secured about the portion of the guiding member 33 which extends therethrough. The proximal portion of the balloon is provided with an elongated skirt 35 with a proximal end (not shown) sealed about the distal end of the hypotubing.

A presently preferred embodiment of the invention, as shown in FIGS. 1-4, is a dilatation catheter for PTCA wherein the outer tubular member 14 has an outer diameter of about 0,51mm (0.02) to about 1,02mm (0.04) (typically about 0,94mm (0.037 inch)), an inner diameter of about 0,38mm (0.015) to about 0,89mm (0.035 inch) (typically about 0,76mm (0.03 inch)). The wall thickness of the outer tubular member 14 can vary from about 0,05mm (0.002) to about 0,20mm (0.008 inch) (typically about 0,08mm (0.003 inch)). The distal inflatable section 12 of the outer tubular member 14 may have an outer diameter of about 0,64mm (0.025) to about 0,76mm (0.030 inch), typically about 0,69mm (0.027 inch) and an inner diameter of about 0,51mm (0.020) to about 0,64mm (0.025 inch), typically about 0,58mm (0.023 inch). The wall thickness will vary depending upon the burst pressure desired but generally will range from about 0,025mm (0.001) to about 0,076mm (0.003 inch). The inner tubular member 15 has an outer diameter of about 0,30mm (0.012) to about 0,41mm (0.016 inch), typically about 0,36mm (0.014 inch). The overall length of the catheter 10 may range from about 100 to about 150 cm but is typically about 135 cm. The length of the inflatable section 12 may range from about 3 to about 30 cm, but typically is about 10 cm. The dimensions of the other embodiments will be similar.

The following is an example of making another presently preferred embodiment of the invention wherein the inflatable member is formed of a zinc polyoleophilic ionomer. In this embodiment pellets of a zinc olefinic ionomer identified as F1855 (a low molecular weight variant of 9020 Surlyn® from E.I. duPont) are extruded at a temperature between about 177°C (350°) to about 232°C (450°F) into tubular stock. Upon exiting from the extrusion die, the tubular stock is quenched in a trough of cool water, preferably about 7-13°C (45-55°F) in order to form a highly amorphous tubular product. The cooled tubular product is stabilized at about 4 to 27°C (40 to 80°F), typically about 16°C (60°F) for about 2 to about 6 hours, typically about 4 hours. The stabilized tubular product is then irradiated at about 45 to about 75 Mrads, preferably about 55 to about 65 Mrads to cross-link the product. The portion of the tubular product which is to be formed into the inflatable member is then heat treated at a temperature of about 107°C (225°F) to about 121°C (250°F) and subjected to an internal pressure of about 3,5bar (50) to about 5,0bar (85 psi), preferably about 4,1bar (60) to about 5,2bar (75 psi), to expand or blow the heat treated portion of the tubular member into a balloon. The balloon is blown slightly larger than the desired inflated size, *e.g*. up to 3.1 mm if an inflated diameter of 3.0 mm is desired, and then it is assembled with other components into a catheter. The balloon is heat treated at about 55°C to about 65°C for about 10 to about 30 minutes to heat shrink the balloon to a diameter the same or slightly larger than its original diameter. Preferably, a heat shrinkable sheath is placed about the balloon during the heat treatment so that small wings are formed. The curve B in Fig. 5 illustrates the outer inflated diameter of a typical inflatable member at various interior pressures. The starting point of curve B, as shown in the drawing, is after the inflatable member has been sufficiently filled with inflation liquid to form a relatively wrinkle free inflatable member. In the pressure range of about 8 atmospheres and extending to about 12 atmospheres, the expansion rate of the inflatable member is relatively high and the expansion mode is elastic. At pressures beyond about 12 atmospheres the expansion of the inflatable member is relatively low, *i.e*. it is relatively noncompliant. Upon deflation of the inflatable member, the diameter of the inflatable member follows the expansion curve shown in the drawing to essentially the starting point with little or no permanent deformation.

In another example, the same extruded and irradiated tubular product described above, which is formed of zinc olefinic ionomer, was treated by heat treating at about 107°C (225°F) to abut 121°C (250°F), but was not inflated at the elevated temperature nor heat shrunk as in the prior example. Curve C in Fig. 5 illustrates typical relationship between the internal fluid pressure and the outer balloon diameter of inflatable members or balloons which have been formed in this manner.

While the graphical relationship depicted by curves A, B and C may at first glance seem disparate, these curves demonstrate the initial elastic expansion within a first pressure range and the relatively little expansion at pressures above the first pressure range. Upon deflation, the outer diameters of the inflatable members follow the same relationship, exhibiting elastic recoil.

A variety of modifications can be made to the present invention. For example, with the aforementioned preferred embodiment only the distal portion of the outer tubular member 14 that was to form the inflatable section 12 was subjected to the heat treatment. If desired, the entire outer tubular member 14 can be given a thermal treatment but the exterior of the non-inflatable section may be provided with an inelastic jacket or coating so that only the inflatable section 12 inflates when subjected to internal pressure. Other modifications include forming the inflatable section of an outer tubular member in accordance with the invention and secure the inflatable section to a catheter shaft of different material or the same material with differing properties. In some instances it may be desirable to inflate the inflatable section before the catheter is introduced into the vascular system of the patient in order to reduce the internal pressure required for the initial expansion of the inflatable section. This preexpansion also decreases the rate of increase of the expansion, but does not substantially change the range of pressure in which the elastic expansion occurs.

## Claims

1. An elongated catheter (10) for delivering a stent comprising:
(a) an elongated catheter shaft (11) having a proximal and distal extremities and an inner lumen (16) extending therein; and
(b) an inflatable element (12) on the distal extremity of the catheter shaft, which has an interior in fluid communication with the inner lumen of the catheter shaft, and which has an inflatable portion which exhibits upon inflation to an internal pressure within a first pressure range a substantial elastic expansion to a distended inflated configuration, and which exhibits within a second pressure range, higher than the first pressure range but at least in part less than about 20 atmospheres, substantially less expansion than the expansion within the first pressure range and which is configured in an uninflated condition to receive an expandable stent on an exterior surface thereof.

2. The catheter of Claim 1, wherein said inflatable element forms very small wings or essentially no wings at all.

3. The catheter of Claim 1 or 2 including an adapter in the proximal extremity of the catheter shaft (11) to direct inflation fluid to the interior of the inflatable element (12) through the inner lumen extending within the catheter shaft.

4. The catheter of Claim 1 or 2 wherein the inflatable element (12) is in a heat shrunk configuration with a diameter less than a diameter formed by inflation in the first pressure range.

5. The catheter of Claim 1 or 2, further comprising a guidewire receiving lumen (17) coextensive at least in part with said inner lumen and extending to a port in the distal end.

6. The catheter of Claim 1 or 2, wherein said inflatable element is formed of a polymeric material.

## Patentansprüche

1. Elongierter Katheter (10) zum Einsetzen eines Stents, welcher aufweist:
(a) einen elongierten Katheterschaft (11) mit einem proximalen und distalen Ende und einem inneren Lumen (16), welches sich darin erstreckt; und
(b) ein aufblasbares Element (12) an dem distalen Ende des Katheterschafts, welches eine Innenseite in Fluidverbindung mit dem inneren Lumen des Katheterschafts aufweist und welches einen aufblasbaren Bereich aufweist, der bei Aufblasen auf einen Innendruck innerhalb eines ersten Druckbereichs eine wesentliche elastische Dehnung zu einer aufgeblähten, aufgeblasenen Konfiguration aufweist, und das in einem zweiten Druckbereich, welcher höher ist als der erste Druckbereich, aber wenigstens teilweise geringer ist als 20 Atmosphären, wesentlich geringere Ausdehnung als die Ausdehnung in dem ersten Druckbereich aufweist, und das in einem nicht aufgeblasenen Zustand so gestaltet ist, dass es einen dehnbaren Stent an einer Außenfläche desselben aufnimmt.

2. Katheter gemäß Anspruch 1, wobei das aufblasbare Element sehr kleine Flügel oder im Wesentlichen gar keine Flügel bildet.

3. Katheter gemäß Anspruch 1 oder 2, der einen Adapter in dem proximalen Ende des Katheterschafts (11) umfasst, um Aufblasfluid zum Innern des aufblasbaren Elements (12) durch das innere Lumen, welches sich in den Katheterschaft erstreckt, zu leiten.

4. Katheter gemäß Anspruch 1 oder 2, wobei das aufblasbare Element (12) in einer wärmegeschrumpften Konfiguration vorliegt mit einem Durchmesser, der geringer ist als der Durchmesser, der bei Aufblasen in dem ersten Druckbereich gebildet wird.

5. Katheter gemäß Anspruch 1 oder 2, welcher weiter ein einen Führungsdraht aufnehmendes Lumen (17) aufweist, welches wenigstens teilweise mit dem inneren Lumen zusammen ausgedehnt ist und sich zu einer Öffnung in dem distalen Ende erstreckt.

6. Katheter gemäß Anspruch 1 oder 2, wobei das aufblasbare Element aus einem Polymermaterial gebildet ist.

## Revendications

1. Cathéter (10) allongé permettant de poser un extenseur *(stent),* comprenant :
(a) un arbre (11) de cathéter allongé ayant une extrémité proximale et une extrémité distale et une lumière interne (16) s'étendant à l'intérieur ; et
(b) un élément gonflable (12) sur l'extrémité distale de l'arbre de cathéter, qui a un intérieur en communication de fluide avec la lumière interne de l'arbre de cathéter et qui a une partie gonflable qui laisse apparaître, après gonflage à une pression interne dans une première plage de pressions, une dilatation sensiblement élastique à une configuration distendue gonflée, et qui laisse apparaître dans une seconde plage de pressions supérieure à la première plage de pressions, mais au moins en partie inférieure à environ 20 atmosphères, sensiblement moins de dilatation que la dilatation dans la première plage de pressions et qui est configuré dans une condition dégonflée pour recevoir un extenseur dilatable sur sa surface externe.

2. Cathéter selon la revendication 1, dans lequel ledit élément gonflable forme de très petites ailes ou essentiellement pas d'ailes du tout.

3. Cathéter selon la revendication 1 ou 2 comprenant un adaptateur dans l'extrémité proximale de l'arbre (11) de cathéter pour diriger le fluide de gonflage de l'élément gonflable (12) à travers la lumière interne s'étendant dans l'arbre de cathéter.

4. Cathéter selon la revendication 1 ou 2, dans lequel l'élément gonflable (12) est dans une configuration thermorétractable avec un diamètre inférieur à un diamètre formé par gonflage dans la première plage de pressions.

5. Cathéter selon la revendication 1 ou 2, comprenant en outre une lumière de réception de fil de guidage (17) coextensive au moins en partie avec ladite lumière interne et s'étendant vers un orifice dans l'extrémité distale.

6. Cathéter selon la revendication 1 ou 2, dans lequel ledit élément gonflable est formé avec un matériau polymère.
